# EUROPÄISCHE PATENTSCHRIFT

(11) **EP 3 414 548 B1**
(45) Veröffentlichungstag und Bekanntmachung des Hinweises auf die Patenterteilung: **07.10.2020**
(21) Anmeldenummer: 17705566.2
(22) Anmeldetag: 09.02.2017
(51) Int. Cl.: G01N 15/06, C12M 1/00

(54) **ZELLVEREINZELUNGSVORRICHTUNG UND VERWENDUNG EINER STRÖMUNGSFORMATION ZUR ZELLVEREINZELUNGSVORRICHTUNG**
CELL SEPARATION DEVICE AND USE OF A FLOW FORMATION FOR THE CELL SEPARATION DEVICE
DISPOSITIF DE SÉPARATION DE CELLULES ET UTILISATION D'UNE FORMATION D'ÉCOULEMENT À DESTINATION DU DISPOSITIF DE SÉPARATION DE CELLULES

(30) Priorität: 12.02.2016 DE 102016202139
(43) Veröffentlichungstag der Anmeldung: 19.12.2018
(73) Patentinhaber: Hamilton Bonaduz AG, 7402 Bonaduz (CH); Fraunhofer-Gesellschaft zur Förderung der angewandten Forschung e.V., 80686 München (DE)
(72) Erfinder: KISSLING, Tom, 4070 Basel (CH); GROSCH, Jens, 4070 Basel (CH); ZUMSTEIN, Thomas, 4070 Basel (CH); TRAUBE, Andrea, 70569 Stuttgart (DE); ETZOLD, Carsten, 7402 Bonaduz (CH); SEILER, Tobias, 7017 Flims Dorf (CH)
(74) Vertreter: Ruttensperger Lachnit Trossin Gomoll
(86) Internationale Anmeldenummer: PCT/EP2017/052812
(87) Internationale Veröffentlichungsnummer: WO 2017/137472

(56) Entgegenhaltungen:
- WO-A1-2008/019964
- WO-A1-2015/158776
- DE-A1-102013 200 927
- DE-U1-202012 002 102

## Beschreibung

Die vorliegende Erfindung betrifft eine Zellvereinzelungsvorrichtung gemäß dem Oberbegriff von Anspruch 1.

Die vorliegende Erfindung betrifft weiter eine Verwendung einer Strömungsformation zur Vereinzelung von lebenden Zellen in einer Zellsuspension.

Eine gattungsgemäße Zellvereinzelungsvorrichtung ist aus der WO 2008/067044 A bekannt.

Grundsätzlich besteht bei Zellkulturen das Problem, dass die nach ihrer Ernte in einer Zellsuspension vorliegenden kultivierten Zellen häufig nicht vollständig vereinzelt vorliegen, sondern zum Teil als Zellcluster oder Zellagglomerationen, in denen mehrere Zellen miteinander verbunden sind.

Diese Zellagglomerationen sind nachteilig, da die darin verbundenen Zellen sich gegenseitig bedecken, so dass eine agglomerierte Zelle - je nach Anzahl der mit ihr verbundenen Zellen - eine unterschiedlich große von der Flüssigkeit der Zellsuspension benetzbare Oberfläche aufweist. Da Zellen Nährstoffe durch die Membran ihrer Zellhülle aufnehmen, haben die Zellen einer Zellagglomeration unterschiedlich guten Zugang zu in der Suspensionsflüssigkeit enthaltenen Nährstoffen. Weiter wird häufig durch den Kontakt der Zellen untereinander das Zellwachstum der miteinander verbundenen Zellen einschränkt, und zwar umso mehr, je größer die Anzahl an kontaktierenden Nachbarzellen einer Zelle ist.

Somit führen Zellcluster oder Zellagglomerationen in Zellsuspensionen aufgrund der dargestellten Mechanismen zu unterschiedlich entwickelten Zellen in der Zellkultur, wobei die Zellagglomerationen in der Regel die Ergiebigkeit der Zellkultur nachteilig beeinflussen. Außerdem steigt die Zellsterblichkeit in Zellagglomerationen, verglichen mit der Zellsterblichkeit von Einzelzellen.

Aus der gattungsgemäßen WO 2008/067044 A ist eine Zellsuspension führende Leitung mit einem Turbulenz-Strömungsabschnitt bekannt, welcher durch Strömungshindernisse in der Leitung gebildet ist. Dabei sind zwei Strömungshindernisse in Strömungsrichtung mit Abstand voneinander angeordnet, die zueinander versetzte durchströmbare Lochmuster aufweisen.

Bei der Durchströmung der beiden Lochmuster in den die Strömungsformation des Standes der Technik bildenden Strömungshindernissen wird ein Turbulenzgrad in der strömenden Zellsuspension erzielt, bei welchem Zellagglomerationen aufgelöst und die agglomerierten Zellen vereinzelt werden.

Nachteilig an der bekannten Strömungsformation der gattungsgemäßen Zellvereinzelungsvorrichtung ist ihr geringer Durchlassquerschnitt, denn zur Erzielung des gewünschten Turbulenzgrads weisen die bekannten Strömungshindernisse in dem am wenigsten obstruktiven Fall nur 50 % des Strömungsquerschnitts der die Zellsuspension führenden Leitung ohne Strömungsformation auf. Das Lochmuster der bekannten Strömungshindernisse kann so weit verengt werden, dass der Durchlassquerschnitt der Strömungshindernisse nur noch 1 % des Strömungsquerschnitts der strömungsführenden Leitung ohne Strömungsformation beträgt.

Eine Vorrichtung zur Zählung von Zellen in einer Zellsuspension mit allen Merkmalen des Oberbegriffs von Anspruch 1 ist aus der DE 10 2013 200 927 A1 bekannt.

Es ist daher Aufgabe der vorliegenden Erfindung, eine technische Lehre anzugeben, welche bei wenigstens gleichem Vereinzelungserfolg und bei gleichem durchströmbaren Leitungsquerschnitt ohne Strömungsformation einen höheren Mengendurchsatz pro Zeiteinheit gestattet, was nachvollziehbarerweise die Wirtschaftlichkeit der Zellvereinzelungsvorrichtung erhöht.

Diese Aufgabe wird erfindungsgemäß durch eine Zellvereinzelungsvorrichtung mit allen Merkmalen des Anspruchs 1 gelöst. In einer solchen Vorrichtung weist die Strömungsformation wenigstens zwei axial hintereinander gelegene Formationsaxialabschnitte auf, von welchen der eine dazu ausgebildet ist, eine die Leitung axial von dem nachfolgend nur als "Behälter" bezeichneten Zellkulturbehälter weg durchströmende Zellsuspension in einem ersten Richtungssinn in Umfangsrichtung zu beschleunigen, und von welchen der jeweils andere dazu ausgebildet ist, die Zellsuspension in einem dem ersten Richtungssinn entgegengesetzten zweiten Richtungssinn in Umfangsrichtung zu beschleunigen.

Gemäß einem weiteren, mit dem vorgenannten durch eine gemeinsame erfinderische Idee verbundenen Erfindungsgedanken wird diese Aufgabe auch gelöst durch die Verwendung einer Strömungsformation mit allen Merkmalen des Anspruchs 11. Gemäß dieser Verwendung erstreckt sich die Strömungsformation längs einer die Strömungsformation zentral durchsetzend gedachten Formationsbahn, wobei die Formationsbahn eine längs der Formationsbahn verlaufende axiale Richtung, eine zur Formationsbahn orthogonale radiale Richtung und eine um die Formationsbahn umlaufende Umfangsrichtung definiert, wobei die Strömungsformation wenigstens zwei axial hintereinander gelegene Formationsaxialabschnitte aufweist, von denen der eine Formationsaxialabschnitt dazu ausgebildet ist, ein den Formationsaxialabschnitt axial anströmendes Fluid in einem ersten Richtungssinn in Umfangsrichtung zu beschleunigen und von welchen der jeweils andere Formationsaxialabschnitt dazu ausgebildet ist, das Fluid in einem dem ersten Richtungssinn entgegengesetzten zweiten Richtungssinn in Umfangsrichtung zu beschleunigen, zur Vereinzelung von lebenden Zellen in einer Zellsuspension als dem strömenden Fluid.

Nachfolgend wird hauptsächlich die Zellvereinzelungsvorrichtung erläutert und weitergebildet, wobei Weiterbildungen der Strömungsformation auch als Weiterbildungen der Verwendung der Strömungsformation verstanden werden sollen, mit der Maßgabe, dass der Begriff der "Leitungsbahn" für die Weiterbildung der Verwendungsansprüche durch den Begriff "Formationsbahn" ersetzt sein soll.

Tatsächlich verlaufen Leitungsbahn und Formationsbahn in der betriebsbereiten Zellvereinzelungsvorrichtung kollinear. Da der Verwendungsanspruch jedoch auf die Verwendung einer Strömungsformation für sich alleine gerichtet ist, wird hier anstelle des leitungsbasierten Koordinatensystems das unmittelbar auf die Strömungsformation bezogene Koordinatensystem der Formationsbahn verwendet.

Erläuterungen der Zellvereinzelungsvorrichtung, die auf die Leitungswandung bezogen sind, sind im Hinblick auf den Verwendungsanspruch als Erläuterungen zu verstehen, die auf den radial äußeren Rand der Strömungsformation gerichtet sind. Dann, wenn sich die Strömungsformation radial bis zur Leitungswand erstreckt, bezeichnet die Radialkoordinate "radial äußerster Rand" der Strömungsformation den gleichen radialen Ort wie die Radialkoordinate "Leitungswand".

Bei der Beschreibung der Zellvereinzelungsvorrichtung unter Umständen genannte Richtungszusätze zur axialen Richtung, etwa zu dem dem Behälter näheren Längsende der Leitung hin oder von dem Behälter weg, sollen für die Verwendung der Strömungsformation unbeachtlich sein. Hier kommt es lediglich auf die durch die Formationsbahn definierte axiale Richtung an, unabhängig von den zwei konkret möglichen axialen Richtungssinnen.

Der Grund für diese Begriffszuordnung zwischen den zur Beschreibung der erfindungsgemäßen Zellvereinzelungsvorrichtung verwendeten Begriffen und dem diesen Begriffen für die Weiterbildung der Verwendung der Strömungsformation zu Grunde zu legenden Verständnis liegt darin, dass die Strömungsformation nicht nur integral mit der jeweiligen Leitung ausgebildet sein muss, sondern auch als gesondertes Formationsbauteil unabhängig von der Leitung vorliegen kann, sodass im letztgenannten Fall das zur Beschreibung der Zellvereinzelungsvorrichtung vorteilhafte leitungsbasierte Koordinatensystem nicht anwendbar ist.

Die Grundidee der vorliegenden Erfindung liegt darin, die axiale Strömung in der Leitung durch die axial hintereinander gelegenen Formationsaxialabschnitte zusätzlich zur axialen Bewegung in entgegengesetzte Umfangsbewegungen zu beschleunigen. Eine Lochblende, wie sie der gattungsgemäße Stand der Technik verwendet, kann dadurch entfallen. Somit kann ein ausreichender Turbulenzgrad ohne die für die Lochblende des Standes der Technik typischen Querschnittsverengungen des Strömungsquerschnitts erreicht werden.

Zwar erreicht die Zellvereinzelungsvorrichtung des Standes der Technik den Turbulenzgrad durch zwei axial aufeinanderfolgende Lochblenden auf axial sehr kurzem Raum, jedoch ist der zwischen Behälter und dem behälterfernen Leitungsende der Leitung zur Verfügung stehende Anordnungsraum in der Regel nicht kritisch, insbesondere da die Leitung nicht gestreckt, also mit geradliniger Leitungsbahn, verlaufen muss, sondern zu einer Wendel, Spirale und dgl. gewickelt sein kann. Die erfindungsgemäße Zellvereinzelungsvorrichtung benötigt daher zwar einen längeren axialen Leitungsabschnitt zur Erzielung eines hohen Turbulenzgrads als die gattungsgemäße Vorrichtung des Standes der Technik, jedoch kann dies mit einer Querschnittsverringerung des Strömungsquerschnitts der Leitung, verglichen mit der identischen Leitung ohne Strömungsformation, erreicht werden, die 15 %, vorzugsweise 10 %, des identischen Leitungsquerschnitts ohne Strömungsformation nicht überschreitet. Damit kann pro Zeiteinheit eine wesentlich größere Menge an Zellsuspension verwirbelt und damit die darin enthaltenen Zellen sicher vereinzelt werden. Die axial geringfügig längere Ausgestaltung der Leitung wird daher durch den erheblich erhöhten Mengendurchsatz pro Zeiteinheit mehr als kompensiert.

Dabei ist daran gedacht, dass axial aufeinanderfolgende Formationsaxialabschnitte dazu ausgebildet sind, die grundsätzlich axial die Leitung durchströmende Zellsuspension in entgegengesetzte Umfangsrichtungen zu beschleunigen. Dadurch wird eine Verwirbelung der Zellsuspension erreicht, die für eine Vereinzelung der darin möglicherweise enthaltenen Zellagglomerate ausreicht. Dies wird außerdem besonders schonend erreicht, was sich vorteilhaft auf die Zellviabilität auswirkt. Viele Zellen sind mechanisch nur schwach belastbar, insbesondere durch in Strömungen häufig auftretende Scherbelastung (Scherspannung). Durch die hier auftretende axial abwechselnde Beschleunigung der Suspensionsströmung in entgegengesetzte Umfangsrichtungen wird ein hoher Turbulenzgrad ohne unerwünscht hohe Scherbelastungen erreicht. Das Vereinzelungsergebnis, beispielsweise bestimmt durch die Anzahl nach Durchgang durch die Strömungsformation in einer vorbestimmten Menge an Zellsuspension enthaltener Zellagglomerate im Vergleich zu deren Anzahl vor Durchgang durch die Strömungsformation, ist hervorragend.

Die Folge der entgegengesetzt gerichteten Umfangsbeschleunigungen kann sein, dass die Zellsuspension, während sie den Turbulenz-Strömungsabschnitt durchströmt, stets im gleichen Richtungssinn, jedoch mit unterschiedlich hohen Geschwindigkeitsbeträgen in Umfangsrichtung strömt. Aus Gründen eines vorteilhaft höheren Turbulenzgrads ist es jedoch bevorzugt, wenn die wenigstens zwei axial hintereinander gelegenen Formationsaxialabschnitte dazu ausgebildet sind, der sie axial durchströmenden Zellsuspension in einem stromaufwärtigen Formationsaxialabschnitt eine in einem ersten Richtungssinn in Umfangsrichtung verlaufende Strömungskomponente zu verleihen, und in einem stromabwärtigen Formationsaxialabschnitt der Zellsuspension eine in einem dem ersten Richtungssinn entgegengesetzten zweiten Richtungssinn in Umfangsrichtung verlaufende Strömungskomponente zu verleihen. In diesem Falle ändert sich die Strömungskomponente der Zellsuspension in Umfangsrichtung nicht nur in ihrem Betrag, sondern auch in ihrem Richtungssinn.

Auch für die Verwendung gilt der zur Erzielung eines besonders hohen Turbulenzgrads in der Zellsuspension bevorzugte Anwendungsfall, dass von den wenigstens zwei Formationsaxialabschnitten der eine Formationsaxialabschnitt dazu ausgebildet ist, dem den Formationsaxialabschnitt axial anströmenden Fluid eine in einem ersten Richtungssinn in Umfangsrichtung verlaufende Strömungskomponente zu verleihen, und dass der jeweils andere Formationsaxialabschnitt dazu ausgebildet ist, dem Fluid eine in einem dem ersten Richtungssinn entgegengesetzten zweiten Richtungssinn in Umfangsrichtung verlaufende Strömungskomponente zu verleihen.

Dabei reicht es aus, wenn das Fluid bzw. die Zellsuspension die Formationsaxialabschnitte auch axial anströmt, also neben der axialen Strömungskomponente noch Strömungskomponenten in andere Strömungsrichtungen: radial oder/und in Umfangsrichtung, aufweist. Die Strömung in der Leitung bzw. durch die Strömungsformation muss an keiner Stelle rein axial sein.

Das Vereinzelungsergebnis kann bei gleichbleibend hoher Zellviabilität verbessert werden, indem die axiale Länge der Strömungsformation und die Anzahl der während dieser axialen Länge stattfindenden Richtungssinnwechsel der Beschleunigung der Strömung in Umfangsrichtung erhöht wird. Gemäß einer vorteilhaften Weiterbildung der Zellvereinzelungsvorrichtung ist daher vorgesehen, dass die Strömungsformation eine Mehrzahl von axial hintereinander gelegenen Formationsaxialabschnitten aufweist, von welchen jeder einem anderen Formationsaxialabschnitt axial nachfolgende Formationsaxialabschnitt dazu ausgebildet ist, die die Leitung in axialer Richtung von dem Behälter weg durchströmende Zellsuspension in einem Richtungssinn in Umfangsrichtung zu beschleunigen, der dem Richtungssinn der Beschleunigung in Umfangsrichtung im axial unmittelbar vorhergehenden Formationsaxialabschnitt entgegengesetzt ist.

Ein Formationsaxialabschnitt im Sinne dieser Anmeldung soll dabei immer ein Axialabschnitt der Strömungsformation sein, in welchem die Suspensionsströmung in Umfangsrichtung in einem einheitlichen Richtungssinn beschleunigt wird. Ändert sich der Richtungssinn der Strömungsbeschleunigung in Umfangsrichtung, beginnt ein neuer Formationsaxialabschnitt, der sich axial so lange erstreckt, bis sich der Richtungssinn der Beschleunigung der Suspensionsströmung in Umfangsrichtung erneut umkehrt.

Wiederum ist aus Gründen eines möglichst hohen erzielbaren Turbulenzgrads bevorzugt, wenn sich durch die Umfangsbeschleunigungen in entgegensetzten Richtungssinnen nicht nur der Betrag der Strömungsgeschwindigkeitskomponente der strömenden Zellsuspension in Umfangsrichtung ändert, sondern der Richtungssinn der Bewegung selbst. Folglich ist es bevorzugt, wenn von der Mehrzahl von axial hintereinander gelegenen Formationsaxialabschnitten jeder einem anderen Formationsaxialabschnitt axial nachfolgende Formationsaxialabschnitt dazu ausgebildet ist, der die Leitung in axialer Richtung von dem Behälter weg durchströmenden Zellsuspension eine in Umfangsrichtung verlaufende Strömungskomponente zu verleihen, deren Umfangsrichtungssinn jenem im axial unmittelbar vorhergehenden Formationsaxialabschnitt entgegengesetzt ist.

Bevorzugt umfasst die Strömungsformation wenigstens acht Formationsaxialabschnitte.

Fachleute wissen üblicherweise, wie eine axiale Strömung in einer Leitung mit einer Strömungskomponente in Umfangsrichtung versehen werden kann, also in Umfangsrichtung beschleunigt bzw. abgelenkt werden kann. Zur Erzielung des erfindungsgemäßen Erfolgs ist jede Art der Strömungsbeeinflussung geeignet, die in axial aufeinanderfolgenden Formationsaxialabschnitten die Zellsuspension in entgegengesetzte Richtungssinne in Umfangsrichtung beschleunigt.

So kann gemäß einer vorteilhaften Weiterbildung der vorliegenden Erfindung konstruktiv die Strömungsformation in wenigstens zwei Formationsaxialabschnitten jeweils wenigstens eine Leitfläche aufweisen, welche derart bezüglich der Leitungsbahn geneigt ist, dass der von der Leitfläche definitionsgemäß weg weisende Normalenvektor der Leitfläche einerseits eine Axialkomponente aufweist, die in axialer Richtung zu dem dem Behälter näheren Längsende der Leitung hin weist, von welchem her die Zellsuspension anströmt, und eine Umfangskomponente aufweist, die in Umfangsrichtung weist. Um die Zellsuspension in axial hintereinandergelegenen Formationsaxialabschnitten in Umfangsrichtung in entgegengesetzte Richtungssinne zu beschleunigen, weisen die Umfangskomponenten der Normalenvektoren von Leitflächen axial hintereinandergelegener Formationsaxialabschnitte in entgegengesetzte Richtungen. Bevorzugt weist die Strömungsformation in mehr als zwei Formationsaxialabschnitten jeweils wenigstens eine derartige Leitfläche auf, besonders bevorzugt in jedem ihrer Formationsaxialabschnitte.

Die Leitfläche kann in einem besonders einfachen Fall eine ebene Fläche sein, welche bezüglich der Leitungsbahn um eine in radiale Richtung verlaufende Anstellachse um einen Anstellwinkel angestellt sein kann. Die Anstellachse kann, muss jedoch nicht, die Leitungsbahn schneiden, sondern kann auch mit Abstand zu dieser verlaufen.

Zusätzlich kann die Leitfläche, insbesondere in dem diskutierten Fall einer ebenen Fläche als Leitfläche, um eine zur Leitungsbahn parallele Drehachse verdreht sein, insbesondere derart, dass der Normalenvektor der Leitfläche auch eine Komponente nach radial innen auf die Leitungsbahn zu aufweist.

Der Anstellwinkel der Leitfläche ist wenigstens am axialen Einströmende eines Formationsaxialabschnitts nicht größer als 45° bezüglich einer die Leitungsbahn enthaltenden Ebene am selben Ort, um den Staudruck am Einströmende eines Formationsaxialabschnitts nicht unnötig zu erhöhen.

Der Anstellwinkel einer Leitfläche in einem Formationsaxialabschnitt kann mit zunehmendem axialen Abstand von dem axialen Einströmende größer werden, sodass die Strömungskomponente in Umfangsrichtung mit zunehmendem axialen Abstand vom Einströmende in dem betreffenden Formationsaxialabschnitt immer größer wird. Die Leitfläche kann dann aus einer Mehrzahl von ebenen Flächenabschnitten gebildet sein, von welchen je zwei in Umfangsrichtung unmittelbar aufeinanderfolgende zueinander abgewinkelt sind. Der Anstellwinkel kann jedoch auch über die axiale Länge eines Formationsaxialabschnitts oder auch über die axiale Länge der gesamten Strömungsformation über alle Leitflächen kontant sein.

Grundsätzlich kann sich die Leitfläche eines Formationsaxialabschnitts ausgehend von einer den Strömungsbereich der Leitung nach radial außen begrenzenden Leitungswand nach radial innen über die Leitungsbahn hinaus erstrecken. Für eine besonders effektive Ablenkung der Suspensionsströmung in Umfangsrichtung ist es jedoch vorteilhaft, wenn wenigstens ein Formationsaxialabschnitt der Strömungsformation wenigstens zwei Leitflächen aufweist. Die beiden Leitflächen sind dann jeweils im selben Axialbereich der Leitung jeweils in einem Radialbereich zwischen Leitungsbahn und Leitungswand, aber in unterschiedlichen Umfangsbereichen angeordnet. Bevorzugt ist eine Mehrzahl von Formationsaxialabschnitten derart ausgebildet, besonders bevorzugt jeder Formationsaxialabschnitt der Strömungsformation.

Um eine in einem Formationsaxialabschnitt möglichst homogene Strömung auszubilden, sind die zwei oder mehr Leitflächen eines Formationsaxialabschnitts bevorzugt bezogen auf die Leitungsbahn derart symmetrisch ausgebildet, dass die eine Leitfläche durch Rotation um die Leitungsbahn in eine jeweils andere Leitfläche desselben Formationsaxialabschnitts überführbar ist.

Dabei gilt: Je mehr Leitflächen in einem Formationsaxialabschnitt in unterschiedlichen Umfangsabschnitten vorgesehen sind, desto sicherer ist einerseits die Beschleunigung der Zellsuspension in Umfangsrichtung, desto größer ist andererseits jedoch auch die Abnahme des durchströmbaren Leitungsquerschnitts, da die Leitflächen als körperliche Gestalten Volumen in dem Leitungsabschnitt beanspruchen, in dem sie angeordnet sind. Bevorzugt sind dabei pro Formationsaxialabschnitt genau zwei oder drei Leitflächen angeordnet, zueinander in Umfangsrichtung versetzt, bevorzugt um 180° (zwei Leitflächen) oder 120° (drei Leitflächen) äquidistant versetzt.

Zur möglichst sanften und damit scherbelastungsarmen Beschleunigung der Zellsuspensionsströmung in Umfangsrichtung kann die wenigstens eine Leitfläche eines Formationsaxialabschnitts wendelförmig ausgebildet sein. Bevorzugt sind alle Leitflächen eines Formationsaxialabschnitts, besonders bevorzugt aller Formationsaxialabschnitte, wendelförmig ausgebildet.

Der Steigungswinkel der wendelförmigen Leitfläche ist durch eine Tangentenebene an die aufgrund ihrer wendelförmigen Ausbildung gekrümmte Leitfläche definiert. Wiederum kann der Steigungswinkel mit größer werdendem axialem Abstand vom axialen Einströmende des Formationsaxialabschnitts zum axialen Ausströmende hin zunehmen. Bevorzugt ist der Steigungswinkel über die gesamte axiale Länge der Leitfläche jedoch konstant. Dann, wenn ein Formationsaxialabschnitt mehr als eine Leitfläche aufweist, sind bevorzugt die Steigungswinkel aller Leitflächen an derselben Axialposition gleich. Vorzugsweise sind die Steigungswinkel einer Leitfläche, insbesondere aller Leitflächen, über die gesamte axiale Länge eines Formationsaxialabschnitts konstant. Bevorzugt sind die Steigungswinkel aller Leitflächen aller Formationsaxialabschnitte betragsmäßig konstant und gleich groß und unterscheiden sich zwischen den unterschiedlichen Formationsaxialabschnitten nur durch ihr unterschiedliches Vorzeichen, um aufgrund ihrer unterschiedlichen Neigung die Beschleunigung der Suspensionsströmung in entgegengesetzte Richtungssinne in Umfangsrichtung leisten zu können.

Grundsätzlich kann die wendelförmige Leitfläche eines Formationsaxialabschnitts die zentral gedachte Leitungsbahn bzw. Formationsbahn beliebig oft umrunden. Für eine besonders effektive und damit wirtschaftliche Strömungsformation ist es jedoch ausreichend und vorteilhaft, wenn die wendelförmige Leitfläche sich längs ihrer Axialerstreckung gerade so weit in Umfangsrichtung um die Leitungsbahn herum erstreckt, dass der sie entlangströmenden Suspension eine eindeutige Beschleunigung oder sogar Bewegung in Umfangsrichtung erteilt wird, bevor die Strömung in den axial nachfolgenden Formationsaxialabschnitt einströmt und dort im entgegengesetzten Richtungssinn in Umfangsrichtung beschleunigt oder sogar bewegt wird. Die Beschleunigung eines Teils der gesamten Zellsuspensionsströmung in Umfangsrichtung reicht dabei aus, etwa 60 % des Massenstroms oder mehr. Versuche haben gezeigt, dass es dabei ausreicht, wenn die wendelförmige Leitfläche längs ihrer axialen Erstreckung wenigstens, vorzugsweise genau, einen Winkel von 180° um die Leitungsbahn überstreicht. Dies gilt bevorzugt für alle Leiflächen ein und desselben Formationsaxialabschnitts. Eine besonders verlustarme Strömung axial durch die Leitung bzw. entlang der Strömungsformation kann bei einer axialen Abfolge von in entgegengesetztem Richtungssinn in Umfangsrichtung beschleunigend wirkenden Formationsaxialabschnitten dadurch erreicht werden, dass der axial einströmseitige Rand einer Leitfläche eines Formationsaxialabschnitts bezüglich des axial ausströmseitigen Randes einer Leitfläche des unmittelbar vorhergehenden (stromaufwärtigen) Formationsaxialabschnitts in Umfangsrichtung versetzt angeordnet ist, vorzugsweise 90° oder 180° versetzt angeordnet ist. Es kann natürlich auch daran gedacht sein, dass die Leitflächen unterschiedlicher axial aufeinanderfolgender Formationsaxialabschnitte randfrei ineinander übergehend verlaufen, wobei dann jedoch bei gleicher axialer Länge der Strömungsanordnung ein weniger starker Turbulenzgrad erreicht wird, was für besonders empfindliche Zellen vorteilhaft sein kann. Die kontinuierliche randfreie Ausbildung einer einzigen Leitstruktur mit unterschiedlichen Leitflächenabschnitten, die den jeweiligen Formationsaxialabschnitten zugeordnet sind, soll jedoch von der vorliegenden Anmeldung nicht ausgeschlossen sein. Grundsätzlich kann weiter daran gedacht sein, die Strömungsformation einstückig mit der Leitung auszubilden, etwa indem die Leitung durch vergleichsweise weichelastischen Silikonkunststoff gebildet ist. Derartiger Silikonkunststoff gestattet nach dem Aushärten eine zerstörungsfreie Entformung eines die Strömungsformation radial innen in der Leitung bildenden Formkerns in axialer Richtung, in welcher die Leitung schließlich auch von der Zellsuspension im Betrieb durchströmt wird. Beispielsweise können die Leitflächen der Formationsaxialabschnitte bei einstückiger Ausbildung der Strömungsformation mit der Leitung von der Leitungswand nach radial innen vorstehen, wobei sie nicht notwendigerweise nach radial innen bis zur Leitungsmitte, also bis zum Ort der virtuellen Leitungsbahn, verlaufen müssen.

Bevorzugt ist die Strömungsformation jedoch an einem von der Leitung gesondert ausgebildeten Formationsbauteil ausgebildet. Dies hat zum einen fertigungstechnische Vorteile, denn ein solches Formationsbauteil kann als gesondertes Bauteil wesentlich einfacher und kostengünstiger mit größerem konstruktiven Freiheitsgrad hergestellt werden als eine einstückig sich radial innerhalb der Leitung erstreckende Strömungsformation.

Ein geeignetes Formationsbauteil ist als Mischerbauteil zur effektiven Vermischung von zwei Komponenten eines Mischerzeugnisses aus der DE 20 2012 002 102 U1 bekannt. Während das Formationsbauteil in der genannten Druckschrift zum Mischen unterschiedlicher flüssiger Komponenten verwendet wird, wird es gemäß der vorliegenden Erfindung zur Trennung von Zellagglomerationen in einer Flüssigkeit verwendet.

Ein weiterer Vorteil in der gesonderten Ausbildung des Formationsbauteils liegt in der erheblich verbesserten Möglichkeit, Leitung und Strömungsformation nach Gebrauch zu reinigen und gegebenenfalls zu sterilisieren, um Leitung und Strömungsbauteil erneut für eine andere Zellsuspension einsetzen zu können, ohne eine Kontamination der Zellsuspension durch die Leitung und die Strömungsformation aufgrund des vorherigen Einsatzes befürchten zu müssen.

Um die Leitung bei vorgegebener Axial-Strömungsgeschwindigkeitskomponente mit langer Verwirbelungsdauer und damit wiederum hohem Vereinzelungserfolg bei gleichzeitig hoher Zellviabilität mit Zellsuspension durchströmen zu können zu können, ist das Formationsbauteil bevorzugt verformbar, sodass es auch in eine baulich lange, nicht geradlinig verlaufende Leitung einsetzbar ist. Bevorzugt ist die Leitung zur Erzielung einer möglichst langen axialen Verwirbelungsstrecke wendelförmig aufgerollt, sodass es für das Formationsbauteil besonders vorteilhaft ist, wenn es biegsam ist, insbesondere um eine oder mehrere mit der Leitungsbahn einen Winkel einschließende Biegeachsen. Der Winkel der Biegeachsen mit der Leitungsbahn ist dabei vorzugsweise ein rechter Winkel.

Bevorzugt ist auch die Leitung verformbar, sodass sie je nach Anwendungsfall in eine geeignete Form gebracht werden kann, jeweils im Rahmen ihrer Verformbarkeit.

Bevorzugt ist das Formationsbauteil als Spritzgussbauteil hergestellt, wobei dann vorteilhafterweise die Verformbarkeit durch die Materialdicke der die Leitflächen aufweisenden Leitstruktur einstellbar ist.

Zur Stabilisierung der Strömungsformation, insbesondere des Formationsbauteils, kann diese eine sich wenigstens längs eines Axialabschnitts der Strömungsformation, vorzugsweise sich axial längs der gesamten Strömungsformation erstreckende Tragstruktur aufweisen, mit welcher die Leitflächen an ihrem radial inneren Ende verbunden sind, vorzugsweise einstückig verbunden sind. Die Tragstruktur ist vorzugsweise eine zentrale Stab- oder Säulenstruktur, mithin eine Seele des Formationsbauteils, deren radiale Abmessung zur Vermeidung unnötiger Strömungshindernisse bevorzugt kleiner ist als die radiale Abmessung der Strömungsformation insgesamt.

Die Zellvereinzelung mit der erfindungsgemäßen Vorrichtung kann vorteilhaft in einem vollständig geschlossenen System erfolgen, ohne dass die Zellsuspension mit der Atmosphäre in Kontakt gelangt. Am stromabwärtigen Ende der Leitung kann ein Auffangbehälter angeordnet sein, in welchen die Zellsuspension mit den vereinzelten Zellen gefördert wird.

Die vorliegende Erfindung wird nachfolgend anhand der beiliegenden Zeichnungen näher erläutert. Es stellt dar:
- Figur 1: eine grobschematische Darstellung durch eine erfindungsgemäße Ausführungsform einer Zellvereinzelungsvorrichtung der vorliegenden Anmeldung im Längsschnitt und
- Figur 2: eine perspektivische Ansicht des in der Zellvereinzelungsvorrichtung von Figur 1 verwendeten Formationsbauteils.

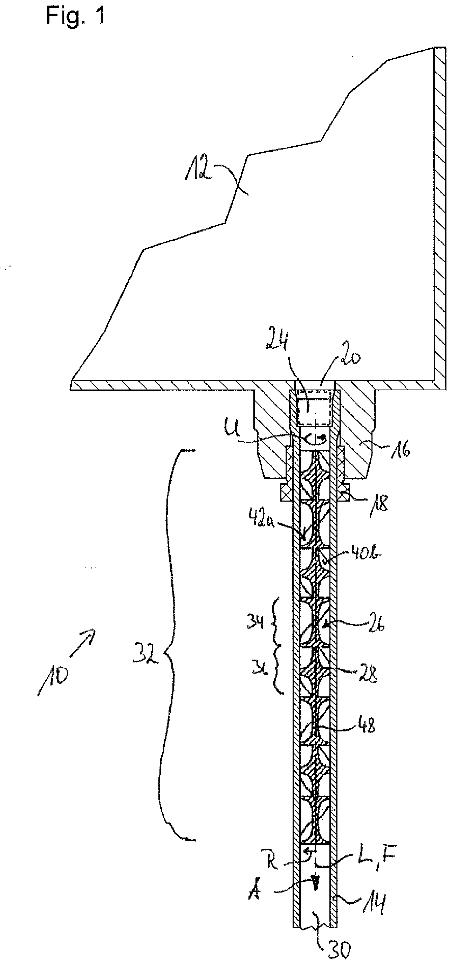

In Figur 1 ist eine erfindungsgemäße Ausführungsform einer Zellvereinzelungsvorrichtung der vorliegenden Anmeldung allgemein mit 10 bezeichnet. Diese umfasst einen Behälter 12, etwa einen Zellkulturbehälter, von welchem in dem grobschematischen Längsschnitt von Figur 1 lediglich ein Eckbereich dargestellt ist.

An den Behälter 12 schließt eine Leitung 14 an, etwa eine flexible Schlauchleitung 14.

Die Schlauchleitung 14 ist an eine Auslassformation 16 des Zellkulturbehälters 12 gekoppelt und dort mittels einer Schraubsicherung 18 positionsgesichert, insbesondere gegen einen Auszug aus der Auslassformation 16.

Die Auslassformation 16 ist in Figur 1 lediglich grobschematisch dargestellt. Im dargestellten Beispiel ist die Leitung 14 mit der Auslassformation 16 dauerhaft gekoppelt. Dies muss nicht so sein. Die Leitung 14 kann auch über an sich bekannte Anschluss- bzw. Kopplungsformationen steckbar und somit schnell verbindbar und lösbar mit der Auslassformation 16 koppelbar sein. Zumindest kann eine solche steckbare Verbindung schneller hergestellt und wieder gelöst werden, als dies durch die Schraubsicherung 18 der Fall ist. Auf die Art und Weise der Strömungsverbindung der Leitung 14 mit einer von der Auslassformation 16 umgebenen Auslassöffnung 20 des Zellkulturbehälters 12 kommt es jedoch vorliegend nicht an.

In dem strichliniert umfassten Bereich 24 in der Anschlussformation 16 und ebenso in der Leitung 14 kann eine in an sich bekannter Weise wirkende Ventilanordnung 24 angeordnet sein, über die eine Strömungsverbindung der Leitung 14 mit der Auslassöffnung 20 wahlweise herstellbar oder sperrbar ist. Die Ventilanordnung 24 kann in an sich bekannter Weise berührungslos durch Magnetfeldmanipulation von außerhalb der Auslassformation 16 schaltbar sein, beispielsweise durch Änderung eines von außerhalb der Auslassformation 16 auf die Ventilanordnung 24 einwirkenden Magnetfelds, wodurch ein ferromagnetischer Ventilkörper zwischen einer Durchlassstellung und einer Sperrstellung verlagerbar sein kann.

Der Zellkulturbehälter 12 dient im vorliegenden Beispiel der Kultivierung adhärenter Zellen, die zunächst an einer Kulturfläche im Zellkulturbehälter 12 anhaftend wachsen und sich nach Zugabe eines entsprechenden Lösemittels in die im Zellbehälter 12 vorhandene Flüssigkeit von ihren Kulturflächen lösen, sodass die Zellen dann frei in der Flüssigkeit im Zellkulturbehälter 12 schweben und somit mit der Flüssigkeit im Zellkulturbehälter 12 eine Zellsuspension bilden. Die in der Zellsuspension gelösten Zellen sind erntereif und werden üblicherweise kurz nach dem Lösen von ihren Kulturflächen aus dem Zellkulturbehälter 12 entnommen, im vorliegenden Beispiel durch die Auslassöffnung 20.

Dabei passiert es in der Regel, dass die Zellen nicht völlig vereinzelt in der Flüssigkeit im Zellkulturbehälter 12 aufgenommen sind, sondern darin Zellagglomerationen aus einer unterschiedlichen Anzahl aneinander anhaftender Zellen bilden. Gewünscht sind jedoch vereinzelte Zellen, da die Zellagglomerationen, wie in der Beschreibungseinleitung dieser Anmeldung geschildert, sich überwiegend nachteilig auf die Entwicklung und die Weiterverarbeitbarkeit der kultivierten Zellen auswirken.

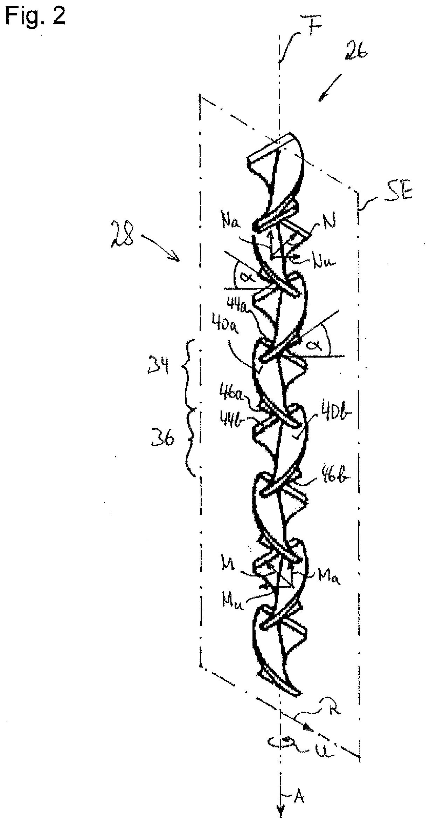

Zum Zwecke der Vereinzelung der Zellen aus den unerwünschten Zellagglomerationen ist in der Leitung 14 eine Strömungsformation 26 angeordnet, im dargestellten Beispiel ausgebildet durch ein gesondert von der Leitung 14 ausgebildetes und in dieser angeordnetes Formationsbauteil 28. Das Formationsbauteil 28 ist in Alleinstellung perspektivisch auch in Figur 2 dargestellt.

Die räumliche Nähe der Strömungsformation 26 zur Auslassöffnung 20 in Figur 1 ist bevorzugt. Die Strömungsformation 26 kann tatsächlich an einer beliebigen Stelle in der Leitung 14 angeordnet sein. Weiterhin können zwischen der Leitung 14 und der Auslassformation 16 weitere, in Figur 1 nicht dargestellte Leitungen zwischenangeordnet sein.

Die Leitung 14, die ebenfalls der Einfachheit halber als geradlinige Leitung 14 dargestellt ist, erstreckt sich längs einer den durchströmbaren Leitungshohlraum 30 zentral durchsetzend gedachten Leitungsbahn L. Da die Strömungsformation 26 in der Leitung 14 kollinear mit dieser angeordnet ist, ist die Leitungsbahn L zugleich auch Formationsbahn F, längs welcher sich die Strömungsformation 26 sowie das Formationsbauteil 28 erstrecken (siehe auch Figur 2).

Die Strömungsformation 26 ist ebenso wie die Leitung 14 flexibel und kann um beliebige Biegeachsen gebogen werden. Sie kann insbesondere raumsparend zu einer Wendel und ähnlichem aufgerollt werden.

Die Strömungsformation 26 dient vorliegend längs ihres Erstreckungsbereichs als Turbulenz-Strömungsabschnitt 32 der Leitung 14, um wenigstens in diesem Turbulenz-Strömungsabschnitt 32 eine die Zellagglomerationen in der sie durchströmenden Zellsuspension vereinzelnde Verwirbelung zu bewirken.

Hierzu weist die Strömungsformation 26 mehrere axial hintereinander angeordnete Formationsaxialabschnitte auf, von denen nachfolgend lediglich die Formationsaxialabschnitte 34 und 36 näher erläutert werden, die in axialer Richtung in der Strömungsformation 26 alternierend angeordnet sind.

Die Leitungsbahn L ebenso wie die Formationsbahn F definieren eine axiale Richtung A. Sie definieren außerdem eine zur axialen Richtung orthogonale radiale Richtung R sowie eine die jeweiligen Bahnen L bzw. F umgebende Umfangsrichtung U.

Der natürliche Richtungssinn der axialen Richtung A ist von dem Zellkulturbehälter 12 weg gerichtet. Der natürliche Richtungssinn der radialen Richtung R ist von der Bahn L bzw. F weg gerichtet und der natürliche Richtungssinn der Umfangsrichtung ist bei Betrachtung in der natürlichen axialen Richtung entgegen dem Uhrzeigersinn.

Die in axialer Richtung A alternierend aufeinanderfolgenden Formationsaxialabschnitte 34 und 36 dienen dazu, eine den Turbulenz-Strömungsabschnitt 32 axial durchströmende Zellsuspension abwechselnd in entgegengesetzte Richtungssinne in Umfangsrichtung U zu beschleunigen.

Die Schnittebene SE, längs welcher die Strömungsformation 26 bzw. das Formationsbauteil 28 in Figur 1 geschnitten dargestellt ist, ist in Figur 2 strichpunktiert dargestellt. Die Schnittebene SE enthält die Formationsbahn F.

Die Formationsaxialabschnitte 34 sind dazu ausgebildet, eine die Leitung 14 bzw. die Strömungsformation 26 in der natürlichen axialen Richtung A durchströmende Zellsuspension in Umfangsrichtung U im Sinne des natürlichen Richtungssinns, also bei Betrachtung in der natürlichen axialen Richtung, im Gegenuhrzeigersinn zu beschleunigen.

Die Formationsaxialabschnitte 36 sind dagegen dazu ausgebildet, die sie axial durchströmende Zellsuspension im entgegengesetzten Richtungssinn in Umfangsrichtung, also bei Betrachtung im natürlichen Richtungssinn der axialen Richtung A im Uhrzeigersinn, zu beschleunigen. Hierzu weisen sowohl die Formationsaxialabschnitte 34 wie auch die Formationsaxialabschnitte 36 Leitflächen 40a bzw. 40b auf. Die Leitflächen 40a und 40b, welche sich jeweils von der Formationsbahn F ausgehend radial nach außen erstrecken, verlaufen wendelförmig mit einander entgegengesetztem Schraubsinn. Tatsächlich weisen sowohl die Formationsaxialabschnitte 34 wie auch die Formationsaxialabschnitte 36 jeweils eine zweite Leitfläche zusätzlich zur Leitfläche 40a bzw. 40b auf, die jedoch in Figur 2 stets vom Betrachter abgewandt ist. Die beiden Leitflächen eines jeden Formationsaxialabschnitts 34 oder 36 sind derart symmetrisch zueinander aufgebaut, dass sie durch Drehung um die Formationsbahn F ineinander überführbar sind, im dargestellten Beispiel jeweils durch Drehung um 180°.

Somit gilt die Beschreibung der Leitflächen 40a und 40b auch für die jeweils zweite Leitfläche desselben Formationsaxialabschnitts 34 bzw. 36. Lediglich in Figur 1 ist ein Teil der zweiten Leitfläche 42a der Formationsaxialabschnitte 34 erkennbar.

Die wendelförmigen Leitflächen 40a, 42a und 40b sowie die nicht erkennbare weitere Leitfläche des Formationsaxialabschnitts 36 weisen über ihre axiale Erstreckung einen konstanten Steigungswinkel α auf, der für beide Formationsaxialabschnitte 34 und 36 betragsmäßig gleich groß und lediglich entgegengesetzt gerichtet ist.

Die wendelförmige Leitfläche 40a weist somit einen Normalenvektor N auf, der eine Axialkomponente Na aufweist, die axial in die Richtung weist, aus der die Leitfläche angeströmt wird, und weist eine Komponente Nu auf, die in Umfangsrichtung in jenem Richtungssinn weist, in dem die auf die Leitfläche 40a auftreffende Strömung beschleunigt wird.

Ebenso weisen die Leitflächen 40b Normalenvektoren M auf, deren Axialkomponente Ma betrags- und richtungsgleich bezüglich der Axialkomponente Na des Normalenvektors N der Leitfläche 40a ist. Weiter ist die Umfangsrichtungskomponente Mu des Normalenvektors M der Leitfläche 40b betragsgleich, aber entgegengesetzt gerichtet wie die Umfangsrichtungskomponente Nu des Normalenvektors N der Leitfläche 40a. Dadurch wird die Beschleunigung der die Formationsaxialabschnitte 34 und 36 durchströmenden Zellsuspension in entgegengesetzte Richtungssinne in Umfangsrichtung U erreicht.

Die Leitflächen ein und desselben Formationsaxialabschnitts erstrecken sich in Umfangsrichtung jeweils um 180° und bilden somit eine halbe Schraubenbasiswindung aus.

Dadurch, dass die beiden Leitflächen ein und desselben Formationsaxialabschnitts um 180° in Umfangsrichtung zueinander versetzt angeordnet sind, bilden sie sowohl einen geradlinigen zur Formationsbahn F orthogonalen axial einströmseitigen Rand 44a für die Formationsaxialabschnitte 34 und 44b für die Formationsaxialabschnitte 36, als auch einen geradlinigen und zur Formationsbahn F orthogonalen axial ausströmseitigen Rand 46a für die Formationsaxialabschnitte 34 und 46b für die Formationsaxialabschnitte 36.

Da sich die Leitflächen 40a, 40b und 42a der Formationsaxialabschnitte 34 bzw. 36 längs ihrer Axialerstreckung um 180° in Umfangsrichtung um die Formationsbahn F bzw. Leitungsbahn L winden, sind die axial einströmseitigen und die axial ausströmseitigen Ränder 44a und 46a bzw. 44b und 46b ein und desselben Formationsaxialabschnitts 34 bzw. 36 zueinander parallel.

Um eine möglichst effektive Verwirbelung der die Leitung 14 mit der Strömungsformation 26 durchströmenden Zellsuspension und damit eine möglichst gründliche Vereinzelung der in der Zellsuspension vorhandenen Zellen zu erreichen, sind vorzugsweise die axial einströmseitigen Ränder 44a bzw. 44b eines ersten Formationsaxialabschnitts 34 oder 36 bezüglich der axial ausströmseitigen Ränder 46b bzw. 46a axial eines dem ersten Formationsaxialabschnitt 34 oder 36 unmittelbar vorausgehenden zweiten Formationsaxialabschnitts 36 oder 34 um 90° zueinander verdreht.

Diese kreuzweise Anordnung der axial aufeinanderfolgenden Formationsaxialabschnitte 34 und 36 ist ohne Stabilitätsverlust des Formationsbauteils 28 möglich, da das Formationsbauteil 28 durch eine zentrale Tragstruktur bzw. Seele 48 stabilisiert ist, die sich im vorliegenden Beispiel über die gesamte axiale Länge der Strömungsformation 26 bzw. des Formationsbauteils 28 erstreckt.

In Figur 1 ist keine Pumpe eingezeichnet, wenngleich eine Förderpumpe zur Erzeugung eines axial die Leitung 14 vom Zellkulturbehälter 12 weg durchströmenden Zellsuspensionsstroms vorhanden sein kann. Allerdings ist eine Pumpe nicht zwingend notwendig. Zusätzlich oder alternativ kann eine Strömung von Zellsuspension durch die Leitung 14 an der Strömungsformation 26 vorbei schwerkraftinduziert sein. Weiter zusätzlich oder alternativ kann die Strömung durch Erzeugung eines Überdrucks in einer Gasblase im Zellkulturbehälter 12 erzeugt werden, sodass der Gasüberdruck im Zellkulturbehälter 12 die Zellsuspension aus dem Behälter 12 über die Auslassöffnung 20 durch die Leitung 14 hindurch austreibt.

Mit der in der vorliegenden Anmeldung beschriebenen Strömungsformation 26 und ihrer Verwendung zur Zellvereinzelung wird ein hervorragendes Vereinzelungsergebnis bei gleichzeitig hoher Zellviabilität erzielt. Je nach "Hartnäckigkeit" der in der Zellsuspension vorhandenen Zellagglomerationen kann der Turbulenz-Strömungsabschnitt 32 durch Hintereinanderanordnung mehrerer Strömungsformationen 26 bzw. mehrerer Formationsbauteile 28 in einer Leitung 14 axial verlängert werden, wodurch die Turbulenzstrecke verlängert wird, die die Zellsuspension durchläuft. So kann durch Anpassung der axialen Länge des Turbulenz-Strömungsabschnitts 32 und durch die Aufwickelbarkeit der Leitung 14 zusammen mit der darin aufgenommenen Strömungsformation 26 jede Zellsuspension ohne großen Bauraumaufwand nahezu vollständig von Zellagglomerationen befreit werden.

Das Formationsbauteil 28 kann formschlüssig, etwa durch ein Zusammenspiel von Vorsprüngen und Ausnehmungen, oder/und stoffschlüssig, etwa durch Klebstoff, in der Leitung 14 axial positionsgesichert sein. Üblicherweise reicht jedoch bereits ein Reibschluss zwischen der Innenwand der Leitung 14 und dem Außenrand des Formationsbauteils 28 aus, um bei der Durchströmung der Leitung 14 eine axiale Verlagerung des Formationsbauteils 28 relativ zur Leitung 14 zu verhindern. Der Reibschluss ist umso ausreichender, je stärker der Leitung 14 gekrümmt angeordnet ist, etwa in der oben beschriebenen Wendelform zur platzsparenden Anordnung auch längerer Leitungsabschnitte.

Sofern im Verständnis der vorliegenden Anmeldung Zweifel bestehen, soll die die Zellsuspension führende Leitung immer als geradlinige Leitung entlang einer geradlinigen Leitungsbahn gedacht sein, wenngleich dies in der Realität technisch nicht zwingend erforderlich ist.

## Patentansprüche

1. Zellvereinzelungsvorrichtung (10) zur Vereinzelung lebender Zellen, umfassend einen Zellkulturbehälter (12) zur Aufnahme einer Zellsuspension und eine mit dem Zellkulturbehälter (12) verbundene Leitung (14) zur Durchleitung von Zellsuspension aus dem Zellkulturbehälter (12), wobei sich die Leitung (14) längs einer die Leitung (14) zentral durchsetzend gedachten Leitungsbahn (L) erstreckt, wobei die Leitungsbahn (L) in der Leitung (14) eine längs der Leitungsbahn (L) verlaufende axiale Richtung (A), eine zur Leitungsbahn (L) orthogonale radiale Richtung (R) und eine um die Leitungsbahn (L) umlaufende Umfangsrichtung (U) definiert, wobei wenigstens ein Abschnitt der Leitung (14) als Turbulenz-Strömungsabschnitt (32) eine eine turbulente Zellsuspensionsströmung erzeugende Strömungsformation (26) aufweist,
**dadurch gekennzeichnet, dass** die Strömungsformation (26) wenigstens zwei axial hintereinander gelegene Formationsaxialabschnitte (34, 36) aufweist, von welchen der eine dazu ausgebildet ist, eine die Leitung (14) axial von dem Zellkulturbehälter (12) weg durchströmende Zellsuspension in einem ersten Richtungssinn in Umfangsrichtung (U) zu beschleunigen, und von welchen der jeweils andere dazu ausgebildet ist, die Zellsuspension in einem dem ersten Richtungssinn entgegengesetzten zweiten Richtungssinn in Umfangsrichtung (U) zu beschleunigen.

2. Zellvereinzelungsvorrichtung (10) nach Anspruch 1,
**dadurch gekennzeichnet, dass** die Strömungsformation (26) eine Mehrzahl von axial hintereinander gelegenen Formationsaxialabschnitten (34, 36) aufweist, von welchen jeder einem anderen Formationsaxialabschnitt (34, 36) axial nachfolgende Formationsaxialabschnitt (34, 36) dazu ausgebildet ist, die die Leitung (14) in axialer Richtung (A) von dem Zellkulturbehälter (12) weg durchströmende Zellsuspension in Umfangsrichtung (U) in einem Umfangsrichtungssinn zu beschleunigen, welcher jenem im axial unmittelbar vorhergehenden Formationsaxialabschnitt (36, 34) entgegengesetzt ist.

3. Zellvereinzelungsvorrichtung (10) nach Anspruch 1 oder 2,
**dadurch gekennzeichnet, dass** die Strömungsformation (26) in wenigstens zwei Formationsaxialabschnitten (34, 36), vorzugsweise in jedem Formationsaxialabschnitt (34, 36), jeweils wenigstens eine Leitfläche (40a, 42a, 40b) aufweist, welche derart bezüglich der Leitungsbahn (L) geneigt ist, dass der Normalenvektor (N, M) der Leitfläche (40a, 42a, 40b) eine Axialkomponente (Na, Ma) aufweist, die in axialer Richtung (A) zu dem dem Zellkulturbehälter (12) näheren Längsende der Leitung (14) hinweist, und eine Umfangskomponente (Nu, Mu) aufweist, die in Umfangsrichtung (U) weist, wobei die Umfangskomponenten (Nu, Mu) von Leitflächen (40a, 42a, 40b) axial hintereinander gelegener Formationsaxialabschnitte (34, 36) in entgegengesetzte Richtungssinne weisen.

4. Zellvereinzelungsvorrichtung (10) nach Anspruch 3,
**dadurch gekennzeichnet, dass** wenigstens ein Formationsaxialabschnitt (34, 36) der Strömungsformation (26), vorzugsweise eine Mehrzahl von Formationsaxialabschnitten (34, 36), besonders bevorzugt jeder Formationsaxialabschnitt (34, 36), jeweils wenigstens zwei Leitflächen (40a, 42a, 40b) aufweist, welche im selben Axialbereich der Leitung (14) in einem Radialbereich zwischen Leitungsbahn (L) und Leitungswand, aber in unterschiedlichen Umfangsbereichen angeordnet sind, wobei bevorzugt die Leitflächen (40a, 42a, 40b) eines Formationsaxialabschnitts (34, 36) bezogen auf die Leitungsbahn (L) derart symmetrisch ausgebildet sind, dass die eine Leitfläche (40a, 42a, 40b) durch Rotation um die Leitungsbahn (L) in eine jeweils andere Leitfläche (40a, 42a, 40b) desselben Formationsaxialabschnitts (34, 36) überführbar ist.

5. Zellvereinzelungsvorrichtung (10) nach Anspruch 3 oder 4,
**dadurch gekennzeichnet, dass** die wenigstens eine Leitfläche (40a, 42a, 40b) wendelförmig ausgebildet ist.

6. Zellvereinzelungsvorrichtung (10) nach Anspruch 5,
**dadurch gekennzeichnet, dass** die wendelförmige Leitfläche (40a, 42a, 40b) längs ihrer axialen Erstreckung wenigstens, vorzugsweise genau, einen Winkel von 180° um die Leitungsbahn (L) überstreicht.

7. Zellvereinzelungsvorrichtung (10) nach einem der Ansprüche 3 bis 6,
**dadurch gekennzeichnet, dass** der axial einströmseitige Rand (44a, 44b) einer Leitfläche (40a, 42a, 40b) eines Formationsaxialabschnitts (34, 36) bezüglich des axial ausströmseitigen Randes (46b, 46a) einer Leitfläche (40a, 42a, 40b) des unmittelbar vorhergehenden Formationsaxialabschnitts (36, 34) in Umfangsrichtung (U) versetzt angeordnet ist, vorzugsweise um 90° oder um 180° versetzt angeordnet ist.

8. Zellvereinzelungsvorrichtung (10) nach einem der vorhergehenden Ansprüche,
**dadurch gekennzeichnet, dass** die Strömungsformation (26) an einem von der Leitung (14) gesonderten Formationsbauteil (28) ausgebildet ist.

9. Zellvereinzelungsvorrichtung (10) nach Anspruch 8,
**dadurch gekennzeichnet, dass** das Formationsbauteil (28) verformbar ist, insbesondere biegsam ist, besonders bevorzugt um eine oder mehrere mit der Leitungsbahn (L) einen Winkel, vorzugsweise einen rechten Winkel, einschließende Biegeachsen.

10. Zellvereinzelungsvorrichtung (10) nach einem der vorhergehenden Ansprüche,
**dadurch gekennzeichnet, dass** der Zellkulturbehälter (12) als Zellkulturbehälter (12) zur Kultivierung adhärenter Zellen eine Kulturfläche aufweist, an welcher die adhärenten Zellen anhaftend wachsen.

11. Zellvereinzelungsvorrichtung (10) nach einem der vorhergehenden Ansprüche,
**dadurch gekennzeichnet, dass** die Strömungsformation (26) frei von Lochblenden ist.

12. Verwendung einer Strömungsformation (26) zur Vereinzelung von lebenden Zellen in einer Zellsuspension als dem strömenden Fluid, welche Strömungsformation (26) sich längs einer die Strömungsformation (26) zentral durchsetzend gedachten Formationsbahn (F) erstreckt, wobei die Formationsbahn (F) eine längs der Formationsbahn (F) verlaufende axiale Richtung (A), eine zur Formationsbahn (F) orthogonale radiale Richtung (R) und eine um die Formationsbahn (F) umlaufende Umfangsrichtung (U) definiert, wobei die Strömungsformation (26) wenigstens zwei axial hintereinander gelegene Formationsaxialabschnitte (34, 36) aufweist, von denen der eine Formationsaxialabschnitt (34, 36) dazu ausgebildet ist, ein den Formationsaxialabschnitt (34, 36) axial anströmendes Fluid in einem ersten Richtungssinn in Umfangsrichtung (U) zu beschleunigen und von welchen der jeweils andere Formationsaxialabschnitt (34, 36) dazu ausgebildet ist, das Fluid in einem dem ersten Richtungssinn entgegengesetzten zweiten Richtungssinn in Umfangsrichtung (U) zu beschleunigen.

## Claims

1. A cell separation apparatus (10) for isolating living cells, encompassing a cell culture container (12) for reception of a cell suspension and a conduit (14) connected to the cell culture container (12) for the conveyance of cell suspension out of the cell culture container (12), the conduit (14) extending along a notional conduit path (L) passing centrally through the conduit (14), the conduit path (L) defining in the conduit (14) an axial direction (A) proceeding along the conduit path (L), a radial direction (R) orthogonal to the conduit path (L), and a circumferential direction (U) proceeding around the conduit path (L), at least a segment of the conduit (14), constituting a turbulent flow segment (32), comprising a flow configuration (26) that generates a turbulent cell suspension flow,
**characterized in that** the flow configuration (26) comprises at least two axial configuration segments (34, 36) located axially behind one another, one of which is embodied to accelerate a cell suspension, flowing through the conduit (14) axially away from the cell culture container (12), in a circumferential direction (U) in a first directional orientation, and the respective other of which is embodied to accelerate the cell suspension in a circumferential direction (U) in a second directional orientation opposite to the first directional orientation.

2. The cell separation apparatus (10) according to Claim 1,
**characterized in that** the flow configuration (26) comprises a plurality of axial configuration segments (34, 36) located axially behind one another, of which each axial configuration segment (34, 36) axially succeeding another axial configuration segment (34, 36) is embodied to accelerate the cell suspension, flowing through the conduit (14) in an axial direction (A) away from the cell culture container (12), in a circumferential direction (U) in a circumferential directional orientation which is directed oppositely from that in the axially immediately preceding axial configuration segment (36, 34).

3. The cell separation apparatus (10) according to Claim 1 or 2, **characterized in that** the flow configuration (26) comprises in at least two axial configuration segments (34, 36), preferably in each axial configuration segment (34, 36), at least one respective directing surface (40a, 42a, 40b) that is inclined with respect to the conduit path (L) in such a way that the normal vector (N, M) of the directing surface (40a, 42a, 40b) has an axial component (Na, Ma) that points in an axial direction (A) toward that longitudinal end of the conduit (14) which is closer to the cell culture container (12), and has a circumferential component (Nu, Mu) that points in a circumferential direction (U), the circumferential components (Nu, Mu) of directing surfaces (40a, 42a, 40b) of axial configuration segments (34, 36) located axially behind one another pointing in opposite directions.

4. The cell separation apparatus (10) according to Claim 3, **characterized in that** at least one axial configuration segment (34, 36) of the flow configuration (26), preferably a plurality of axial configuration segments (34, 36), particularly preferably each axial configuration segment (34, 36), respectively comprises at least two directing surfaces (40a, 42a, 40b) that are arranged in the same axial region of the conduit (14) in a radial region between the conduit path (L) and conduit wall but in different circumferential regions, the directing surfaces (40a, 42a, 40b) of an axial configuration segment (34, 36) preferably being embodied symmetrically with respect to the conduit path (L) in such a way that the one directing surface (40a, 42b, 40b) is transformable, by rotation around the conduit path (L), into a respective other directing surface (40a, 42a, 40b) of the same axial configuration segment (34, 36).

5. The cell separation apparatus (10) according to Claim 3 or 4, **characterized in that** the at least one directing surface (40a, 42a, 40b) is embodied helically.

6. The cell separation apparatus (10) according to Claim 5,
**characterized in that** the helical directing surface (40a, 42a, 40b) sweeps out along its axial extent at least, preferably exactly, an angle of 180° around the conduit path (L).

7. The cell separation apparatus (10) according to one of Claims 3 to 6,
**characterized in that** the axially inflow-side edge (44a, 44b) of a directing surface (40a, 42a, 40b) of one axial configuration segment (34, 36) is arranged with an offset, preferably arranged with a 90° or 180° offset, in a circumferential direction (U) with respect to the axially outflow-side edge (46b, 46a) of a directing surface (40a, 42a, 40b) of the immediately preceding axial configuration segment (36, 34).

8. The cell separation apparatus (10) according to one of the preceding claims,
**characterized in that** the flow configuration (26) is embodied on a configuration component (28) separate from the conduit (14).

9. The cell separation apparatus (10) according to Claim 8,
**characterized in that** the configuration component (28) is deformable, in particular is flexible, particularly preferably around one or several flexure axes that enclose an angle, preferably a right angle, with the conduit path (L).

10. The cell separation apparatus (10) according to one of the preceding claims,
**characterized in that** the cell culture container (12), as cell culture container (12) for cultivating adherent cells, comprises a culture surface, on which the adherent cells grow adhering.

11. The cell separation apparatus (10) according to one of the preceding claims,
**characterized in that** the flow configuration (26) is free of perforated sheets.

12. Use of a flow configuration (26) to isolate living cells in a cell suspension constituting the flowing fluid, which flow configuration (26) extends along a notional configuration path (F) passing centrally through the flow configuration (26), the configuration path (F) defining an axial direction (A) proceeding along the configuration path (F), a radial direction (R) orthogonal to the configuration path (F), and a circumferential direction (U) proceeding around the configuration path (F), the flow configuration (26) comprising at least two axial configuration segments (34, 36), located axially behind one another, of which the one axial configuration segment (34, 36) is embodied to accelerate a flow of fluid, impinging axially on the axial configuration segment (34, 36) in a circumferential direction (U) in a first directional orientation, and of which the respective other axial configuration segment (34, 36) is embodied to accelerate the fluid in a circumferential direction (U) in a second directional orientation opposite to the first directional orientation.

## Revendications

1. Dispositif de séparation de cellules (10) pour séparer des cellules vivantes, comprenant un récipient de culture cellulaire (12) pour recevoir une suspension de cellules et un conduit (14) relié au récipient de culture cellulaire (12) pour faire passer la suspension de cellules du récipient de culture cellulaire (12), dans lequel le conduit (14) s'étend le long d'un trajet de conduit (L) pensé traversant de manière centrale le conduit (14), le trajet de conduit (L) définissant dans le conduit (14) une direction axiale (A) s'étendant le long du trajet de conduit (L), une direction radiale (R) orthogonale au trajet de conduit (L) et une direction circonférentielle (U) s'étendant autour du trajet de conduit (L), dans lequel au moins une section du conduit (14) présente, en tant que section d'écoulement turbulent (32), une formation d'écoulement (26) générant un écoulement de suspension cellulaire turbulent,
**caractérisé en ce que** la formation d'écoulement (26) présente au moins deux sections axiales de formation (34, 36) se succédant axialement, dont l'une est adaptée pour accélérer une suspension cellulaire s'écoulant axialement dans le conduit (14) en s'éloignant du récipient de culture cellulaire (12) dans une première direction dans la direction circonférentielle (U), et dont l'autre est adaptée pour accélérer la suspension cellulaire dans une deuxième direction opposée à la première direction dans la direction circonférentielle (U).

2. Dispositif de séparation de cellules (10) selon la revendication 1,
**caractérisé en ce que** la formation d'écoulement (26) comprend une pluralité de sections axiales de formation (34, 36) se succédant axialement, dont chaque section axiale de formation (34, 36) suivant axialement une autre section axiale de formation (34, 36) est adaptée pour accélérer la suspension cellulaire s'écoulant dans le conduit (14) dans la direction axiale (A) en s'éloignant du récipient de culture cellulaire (12) dans la direction circonférentielle (U) dans un sens circonférentiel opposée à celle de la section axiale de formation immédiatement précédente (36, 34).

3. Dispositif de séparation de cellules (10) selon la revendication 1 ou 2,
**caractérisé en ce que** la formation d'écoulement (26) présente dans au moins deux sections de formation axiales (34, 36), de préférence dans chaque section de formation axiale (34, 36), respectivement au moins une surface de guidage (40a, 42a, 40b) qui est inclinée par rapport au trajet de conduit (L) de telle sorte que le vecteur normal (N, M) de la surface de guidage (40a, 42a, 40b) présente une composante axiale (Na, Ma) orientée dans la direction axiale (A) vers l'extrémité longitudinale du conduit (14) plus proche du récipient de culture cellulaire (12), et une composante circonférentielle (Nu, Mu) orientée dans la direction circonférentielle (U), les composantes circonférentielles (Nu, Mu) des surfaces de guidage (40a, 42a, 40b) des sections axiales de formation se succédant axialement (34, 36) et étant orientées dans des sens de directions opposées.

4. Dispositif de séparation de cellules (10) selon la revendication 3,
**caractérisé en ce qu'**au moins une section axiale de formation (34, 36) de la formation d'écoulement (26), de préférence une pluralité de sections axiales de formation (34, 36), de manière particulièrement préférée chaque section axiale de formation (34, 36), présente respectivement au moins deux surfaces de guidage (40a, 42a, 40b) qui sont disposées dans la même zone axiale du conduit (14) dans une zone radiale entre le trajet de conduit (L) et la paroi de conduit, mais sont disposées dans différentes zones circonférentielles, les surfaces de guidage (40a, 42a, 40b) d'une section axiale de formation (34, 36) étant de préférence formées symétriquement par rapport au trajet de conduit (L) de telle sorte qu'une surface de guidage (40a, 42a, 40b) peut être transférée dans une autre surface de guidage (40a, 42a, 40b) de la même section axiale de formation (34, 36) par rotation autour du trajet de conduit (L).

5. Dispositif de séparation de cellules (10) selon la revendication 3 ou 4,
**caractérisé en ce que** ladite au moins une surface de guidage (40a, 42a, 30 40b) est formée en hélice.

6. Dispositif de séparation de cellules (10) selon la revendication 5,
**caractérisé en ce que** la surface de guidage hélicoïdale (40a, 42a, 40b) balaye le long de son extension axiale au moins, de préférence avec précision, un angle de 180° autour du trajet de conduit (L).

7. Dispositif de séparation de cellules (10) selon l'une des revendications 3 à 6,
**caractérisé en ce que** le bord côté entrée axiale (44a, 44b) d'une surface de guidage (40a, 42a, 40b) d'une section axiale de formation (34, 36) est disposé décalé dans la direction circonférentielle (U) par rapport au bord côté sortie axiale (46b, 46a) d'une surface de guidage (40a, 42a, 40b) de la section axiale de formation (36, 34) immédiatement précédente, de préférence décalé de 90° ou de 180°.

8. Dispositif de séparation de cellules (10) selon l'une des revendications précédentes,
**caractérisé en ce que** la formation d'écoulement (26) est formée sur un élément de formation (28) séparé du conduit (14).

9. Dispositif de séparation de cellules (10) selon la revendication 8,
**caractérisé en ce que** l'élément de formation (28) est déformable, en particulier flexible, de préférence autour d'un ou plusieurs axes de flexion formant un angle, de préférence un angle droit, avec le trajet de conduit (L).

10. Dispositif de séparation de cellules (10) selon l'une des revendications précédentes,
**caractérisé en ce que** le récipient de culture cellulaire (12), en tant que récipient de culture cellulaire (12) pour la culture de cellules adhérentes, présente une surface de culture sur laquelle les cellules adhérentes croissent de manière adhérente.

11. Dispositif de séparation de cellules (10) selon l'une des revendications précédentes,
**caractérisé en ce que** la formation d'écoulement (26) est exempte de trous d'épingle.

12. Utilisation d'une formation d'écoulement (26) pour la séparation de cellules vivantes dans une suspension cellulaire comme fluide d'écoulement, laquelle formation d'écoulement (26) s'étend le long d'un chemin de formation (F) traversant de manière centrale la formation d'écoulement (26), dans lequel le chemin de formation (F) définit une direction axiale (A) s'étendant le long du chemin de formation (F), une direction radiale (R) orthogonale au chemin de formation (F), et une direction circonférentielle (U) s'étendant autour du chemin de formation (F), dans lequel la formation d'écoulement (26) comprend au moins deux sections axiales de formation (34, 36) situées axialement l'une derrière l'autre, dont une section axiale de formation (34, 36) est adaptée pour accélérer un fluide s'écoulant axialement contre la section axiale de formation (34, 36) dans une première direction dans la direction circonférentielle (U) et dont l'autre section axiale de formation respective (34, 36) est adaptée pour accélérer le fluide dans une deuxième direction opposée à la première direction dans la direction circonférentielle (U).
